# EUROPÄISCHE PATENTSCHRIFT

(11) **EP 0 984 041 B1**
(45) Veröffentlichungstag und Bekanntmachung des Hinweises auf die Patenterteilung: **14.04.2004**
(21) Anmeldenummer: 99117357.6
(22) Anmeldetag: 03.09.1999
(51) Int. Cl.: C07D 487/22, C09B 47/06, C09B 69/10, C08K 5/3467, A61L 2/00, C02F 1/50

(54) **Metallierte oder unmetallierte Phthalocyanine**
Metallised or metal-free phthalocyanine
Phtalocyanine métallisée ou non metallisée

(30) Priorität: 05.09.1998 DE 19840608
(43) Veröffentlichungstag der Anmeldung: 08.03.2000
(73) Patentinhaber: Deutsches Zentrum für Luft- und Raumfahrt e.V, 53127 Bonn (DE)
(72) Erfinder: Funken, Karl-Heinz, Dr., 53225 Bonn (DE); Faust, Delia, 53783 Eitorf-Hove (DE); Ortner, Jürgen, Dr., 51065 Köln (DE); Sattler, Christian, Dr., 53111 Bonn (DE)
(74) Vertreter: Jönsson, Hans-Peter, Dr.Dipl.-Chem.

(56) Entgegenhaltungen:
- EP-A- 0 003 371
- EP-A- 0 047 716
- EP-A- 0 345 171
- EP-A- 0 891 977
- WO-A-90/06963
- GB-A- 2 260 996
- US-A- 4 986 921
- MOUGANG HU ET AL: "hydroxyphthalocyanines as potential.." JOURNAL OF MEDICINAL CHEMISTRY, Bd. 41, Nr. 11, 1998, Seiten 1789-1802, XP002174054 AMERICAN CHEMICAL SOCIETY. WASHINGTON.; US

## Beschreibung

Gegenstand der Erfindung sind metallierte oder unmetallierte Phthalocyanine sowie ein Verfahren zu ihrer Herstellung sowie deren Verwendung. Weiterhin sind Gegenstand der vorliegenden Erfindung Photosensibilisatoren umfassend die genannten Phthalocyanine soweit die Verwendung der Photosensibilisatoren zur Initiierung photochemischer Reaktionen, insbesondere zur katalytischen Bildung von Singulett-Sauerstoff.

Die Erfindung betrifft insbesondere oberflächengeladene Phthalocyanine, welche kovalent an einen polymeren Werkstoff gebunden sind.

Hydrophile polymergebundene Phthalocyanine besitzen vorteilhafte Eigenschaften als Photosensibilisatoren. Diese Vorteile kommen insbesondere an einer Grenzschicht Polymer - Wasser zur Geltung, da mit diesen neuartigen Stoffen auch ansonsten hydrophobe Polymeroberflächen in einen ausreichend intensiven Kontakt zu einer wäßrigen Phase gebracht werden können. So lassen sich photochemische Reaktionen initiieren, die ansonsten nicht ablaufen würden. Für die wasserlöslichen polymergebundenen Phthalocyanine gibt es potentiell andere Anwendungsbiete bzw. Betriebsweisen als für die wasserunlöslichen hydrophilen Polymere.

Unmetallierte Phthalocyanine und auch metallierte Phthalocyanine können als effiziente Photosensibilisatoren genutzt werden. Sie werden bei der Bestrahlung mit Licht ausreichender Wellenlänge elektronisch angeregt. In geeigneter Kombination mit chemischen Reaktionspartnem können die angeregten Sensibilisatoren entweder die Energie oder eine Ladung auf den betreffenden Reaktionspartner übertragen, welcher in nachfolgenden Reaktionen abreagiert. Als Akzeptor der Energie kommt eine Vielzahl anderer Spezies in Frage. Auch können elektronisch angeregte Phthalocyanine sowohl als Donatoren wie auch als Akzeptoren unter Bildung von Exciplexen fungieren, die dann zu chemischen Reaktionen führen können (T. Mashiko, D. Dolphin, Comprehensive Coord. Chem. 2, 1987, 813-898) in bezug auf Porphyrine.

Von anwendungstechnischem Interesse sind z.B. sensibilisierte Photooxygenierungen, sensibilisierte Photoisomerisierungen, sensibilisierte Photocycloadditionen und sensibilisierte Photodesulfonylierungen. Die sensibilisierte Aktivierung von Sauerstoff, der im Grundzustand als Triplett-Sauerstoff vorliegt und sensibilisiert in Singulett-Sauerstoff überführt werden kann, ist besonders bedeutsam. Singulett-Sauerstoff besitzt eine vom Triplett-Sauerstoff verschiedene Reaktivität. In vielen praktischen Fällen wird der Sensibilisator im Reaktionsgemisch homogen gelöst. Dabei ist es jedoch von Nachteil, daß der Sensibilisator nach erfolgter Reaktion bei der Aufarbeitung des Reaktionsgemischs häufig mit hohem Aufwand abgetrennt und ggf. recycliert werden muß. Außerdem wird oft gefordert, daß das Produkt keine Sensibilisatorreste mehr enthalten darf. Ein an sich bekannter Weg zur Verminderung des Aufwandes besteht darin, den Sensibilisator an Oberflächen von Trä-germaterialien entweder physikalisch (durch Adsorption) oder chemisch (durch kovalente, koordinative oder elektrostatische Bindung) zu fixieren. Er kann dann nach erfolgter Reaktion z.B. durch Filtration leicht abgetrennt und wiederverwendet werden. In der Literatur werden Beispiele zur Verwendung von Sensibilisatoren und zu ihrer Heterogenisierung beschrieben (A.M. Braun, M.-T. Maurette, E. Oliveros, Photochemical Technology, John Wiley & Sons, Chichester 1991; H. Böttcher (Ed.), Technical Applications of Photochemistry, Deutscher Verlag für Grundstoffindustrie 1991).

Die Adsorption von Rose Bengale an Kieselgel beschreiben S. Tamagaki et al. (J. Org. Chem. 45, 1573, 1980).

W. Wolters beschreibt in DE 3836759 A1 und DE 3924815 A1 die Verwendung von Phthalocyaninen und diazotiertem b-Naphthol, physikalisch gebunden, das mit einem Bindemittel als Katalysator zur Aktivierung von Sauerstoff fixiert ist. Als Bindemittel werden einerseits Acrylsäureester und/oder Methacrylsäureester und andererseits Polyurethane verwendet.

Zhong-Wie Gu, John D. Spikes, Pavla Kopeckova und Jindrich Kopeck J. Pharm. Sci., EN, 83 (1994) 11, 1608-1616 berichten über ein polymergebundenes Phthalocynin, welches aus der Aminolyse von Zink(II)-4,9,16,23-tetraaminophthalocyanin- und N(2-hydroxypropyl)methacrylamid entsteht.

Kousuke Kusuda, Taiji Kanda und Keizo Tanisaka Ber. Bunsenges. Phys. Chem. 96 (1992) 8, 998-1004, beschreiben in ihrem Artikel die Fixierung von symmetrischem Tetra-sulfophthalocyanin an Aluminiumoxid, Siliciumoxid, Holzkohle und kationischem Latex. Außerdem zitieren sie die Fixierung dieser Verbindung als Salz an Polyvinylaminen, Polylysin und polyquarternären Ammoniumsalzen. Sie beschreiben auch die Fixierung von symmetrischem Tetracarbonsäurephthalocyaninderivaten an Hyflo Super-Cel, einem Kieselgelderivat.

Raymond Bonnett et al. (WO 93/00815) berichten über die Fixierung von Tetrabutylphthalocyanin an Cellulose.

H. Eichhorn et al. (Macromol. Chem. Phys. 196, 115, 1995) berichten über die Verwendung von symmetrisch substituierten Methacryloylphthalocyaninen, die vierfach in Methacrylpolymere eingebunden sind.

Hirofusa Shirai et al, US-Patent 5,053,464, beschreiben in ihrem Patent die Polymerisierung von veresterten Phthalocyaninen zu Polyurethanen. Dabei wird von einer Carboxygruppe am Phthalocyanin ausgegangen.

Stephen F. Yates et al., US-Patent 4,986,921, beschreiben die gleiche Vorgehensweise wie oben für Cellulose.

Im Stand der Technik sind eine Reihe von Verfahren zur Desinfektion und/oder Detoxifikation von Wasser und/oder Wasser enthaltenen Umgebungen sowie von Gegenständen bekannt.

An erster Stelle sei hier bekanntermaßen die Chlorierung genannt. Siehe hierzu beispielsweise Dugan A.M. 1996, The products of water chlorination as inducers of gene mutations, Tsitol. Genet. 30: 76 - 81; Craun, G.F. 1988, Surface water supplies and health, J. Am. Water Works Assoc.: 80: 40 - 42; Singh, A. Yeager R., and McFeters G.A. 1986a, Assessment of in vivo revival, growth and pathogenicity of E. coli strains after copper- and chlorine-induced injury, Appl. Environ. Microbiol. 52: 832 - 837; Zierler, S., Danly R.A., Feingold L., 1986, Type of disinfectant in drinking water and patterns of mortality in Massachusetts, Environ. Health Perspect 69: 275 - 279; Jolley, R.L., Brungs W.A., and Cummings R.B., Eds. 1985, Water chlorination: Chemistry, Environmental Impact, and Health Effects, Lewis Pubs., Chelsea, MI; Tuthill, R. W., Giusti R.A., Moore G.S., Calabrese E. J. 1982, Health effects among newborns after prenatal exposure to ClO₂-disinfected drinking water. Environ. Health Perspect 46: 39 - 45. Der wesentliche Nachteil der Chlorierung besteht in den hohen Kosten durch permanenten Chemikalienverbrauch. Darüber hinaus werden bekanntermaßen giftige, beispielsweise kanzerogene oder mutagene Nebenprodukte, wie Trihalomethane und andere organische chemische Verbindungen gebildet. Weiterhin beeinträchtigt der unangenehme Geruch und der Geschmack das Wasser. Bekanntermaßen sind einige Mikroorganismen resistent gegen Chlor, so daß häufig noch eine ergänzende Behandlung angeschlossen werden muß. Viele Mikroorganismen werden durch Chlor zwar in ihrer Reproduktionsfähigkeit gehindert, sind jedoch trotzdem in der Lage, Enterotoxine zu produzieren.

Neben der Chlorierung ist die Chloraminierung, beispielsweise aus Sobsey, M.D. 1989, Inactivation of health-related microorganisms in water by disinfection processes, Water Sci. Technol. 21: 179 - 195; Groethe, D. R. und Eaton J. G. 1975, Chlorine induced mortality in fish. Trans. Am Fish Soc. 104: 800 - 805 bekannt. Auch hier besteht ein wesentlicher Nachteil in den hohen Kosten durch permanenten Chemikalienverbrauch. Wenn auch hier keine Trihalomethane gebildet werden, so ist doch die Chloraminierung nicht so effektiv wie die Chlorierung. Ein unangenehmer Geschmack ist auch hier bei der Detoxifizierung und Desinfektion von Wasser zu beobachten. Viele Organismen sind resistent gegen die Chloranimierung, so daß auch hier zusätzliche Maßnahmen erforderlich sind. Besonders hervorzuheben ist, daß die Chloranimierung sehr schädlich für aquatische Systeme ist. Mutagene und kanzerogene Effekte auf den Menschen werden vermutet.

Die Wasserbehandlung mit Chlordioxid ist beispielsweise aus Berg, J.D., Roberts P.V., and Martin A. 1986, Effect of chlorine dioxide on selected Membrane functions of Escherischia coli. J. Appl. Bacteriol. 60: 213 - 220 bekannt. Chlordioxid muß vor Gebrauch stets neu gebildet werden, da es keine Möglichkeit der Lagerung gibt. Dementsprechend ist das Verfahren wesentlich teurer als die Behandlung des Wassers mit Chlor. Eine Bildung von Trihalomethanen und Chloramin ist nicht zu befürchten, jedoch führen die Nebenprodukte Chlorit und Chlorat zur Methämoglobinämie, da sich diese auf das Hämoglobin des Menschen auswirken können.

Die Ozonisierung gemäß Kir'ianova, E.V. 1996, Optimization of conditions of ozone water disinfection by the method of mathematical planning of an experiment. Gig. Sanit. 4: 3-6; Farooq, S., Akhlaque S. 1983, Comparative response of mixed cultures of bacteria and viruses to ozonisation. Water Res. 17: 809 - 812 ist in Bezug auf die Konstruktion, den Betrieb und die Wartung sehr aufwendig. Da Ozon keine stabile Verbindung ist, muß das Wasser zur längeren Haltbarkeit meist noch mit Chlor versetzt werden. Auch gegen Ozon sind einige Mikroorganismen resistent. Als Nebenprodukte bilden sich häufig Aldehyde, die mutagen wirken können.

Ein weiteres Verfahren zur Behandlung von Wasser und/oder Wasser enthaltenen Umgebungen besteht in der Bestrahlung mit UV-Licht. Siehe hierzu beispielsweise Harris, M. G., Buttino L.M., Chang J.C., Wan M. 1993, Effects of ultraviolet radiation on contact lense parameters , Optom. Vis. Sci 70: 739 - 742; Martiny, H., Wlodavezyk K., Harms G., Ruden H. 1988, The use of UV rays for the disinfection of water. I. Microbiologic studies of drinking water. Zentralbl. Bakteriol. Mikrobiol. Hag. (B). 185: 350 - 367; Harris, G.V., Adams V.D., Sorensen D.L., and Dupont R.R. 1987, The influence of photoreactivation and water quality on ultraviolet disinfection of secondary municipal wastewater. J. Water Pollut. Control Fed. 59: 781 - 787; Muraca, P., Stout J.E., Yu V.L. 1987, Comparative assessment of chlorine, heat, ozone, an UV-light for killing Legionella pneumophila within a model plumbing system, Appl. Environ. Microbiol. 53: 447 - 453; Myhrstadt, J.A., 1979, Disinfection of sewage water by ultraviolet irradiation. NIPH, Ann 2: 11 - 16. Da hier keine aktiven Substanzen in behandeltem Wasser verbleiben, muß in der Regel nachträglich chloriert werden, wodurch wiederum zusätzliche Kosten entstehen. In Abhängigkeit von der Trübung, dem Sauerstoffgehalt, dem Anteil und der Art gelöster Teilchen und chemischer Verunreinigungen des zu behandelnden Wassers ist die Dosisbestimmung sehr problematisch. Es besteht die Möglichkeit der Photoreaktivierung; es muß daher sichergestellt werden, daß das Wasser nicht während oder nach der UV-Bestrahlung an Licht aufbewahrt wird.

Durch Biofilmbildung auf der Lampe ist eine ständige Überwachung und sorgfältige Reinigung der Lampe - chemisch, mechanisch oder mit Ultraschall - wichtig, wodurch zusätzliche Wartungskosten entstehen. Beim Betrieb der Lampe ist auf einen ausreichenden Personenschutz zu achten, da das UV-Licht bekanntermaßen mutagen und kanzerogen wirkt. Durch UV-Licht ermüden eine Reihe von Materialien. Bekanntermaßen wird durch UV-Licht die Bildung von gesundheitsschädlichem Ozon gefördert.

Aus Ahlstrom, S.B. and Lessel T., 1986, Irradiation of municipal sludge for pathogen control. In: Control of Sludge Pathogens, C.A. Sorber, Ed. Water Pollution Control Fedration, Washington D.C. ist die Behandlung von Wasser mit Gamma-Strahlung und hochenergetischer Elektronenstrahlung bekannt. Hierbei handelt es sich um sehr teure aufwendige Anlagen, die ein hohes Sicherheitsrisiko beinhalten. Die Wirkung kann verbessert werden, wenn zusätzlich noch Hitze oder Sauerstoff zugeführt wird, wodurch sich jedoch die Kosten weiterhin erhöhen.

Aus Polyzois, G.L., Zissis A. J., Yannikakis S.A. 1995. The effect of glutaraldehyde and microwave disinfection on some properties of denture resin. Int. J. Prosthodont 8: 150 - 154; und Niederwohrmeier, B., Bohm R., Strauch D. 1985, Microwave treatment as an alternative pasteurization process for the disinfection of sewage sludge: Experiments with the treatment of liquid manure, pp. 135-147 in: Inactivation of Microorganisms in Sewage Sludge by Stabilisation Processes, D. Strauch, A.H. Havelaar, and O.L. L'Hermite, Eds. Elsevier Applied Science, London ist die Behandlung von Wasser mit Mikrowellenstrahlung bekannt. Auch diese Maßnahme ist sehr teuer, da aufwendige Anlagen und Sicherheitsmaßnahmen erforderlich sind. Durch die zusätzliche Wärmezufuhr werden die Kosten weiterhin erhöht, wobei jedoch die Wirkung der Strahlung verbessert ist. Für große Volumina ist die Mikrowellenstrahlung jedoch sehr problematisch, da eine optimale Durchmischung gewährleistet sein sollte.

Aus Joyce, T.M., McGuigan K.G., Elmore-Meegan M., Conroy R.M. Inactivation of fecal bacteria in drinking water by solar heating, Appl. Environ. Microbiol. 62: 399 - 402 und Fuhrmann, H., Floerke I., Bohm K.H. 1986, The problem of heat activation of bacterial spores after disinfection with regard to an aerosol method of decontaminating equipment and rooms. Zentralbl. Bakteriol. Mikrobiol. Hyg. (B) 182: 515 - 524 ist die Hitzesterilisation bekannt. Auch diese ist mit einem hohen Energieaufwand verbunden, wobei nicht immer eine vollständige Inaktivierung aller Organismen erreicht wird. Auch ist die Hitzesterilisation nicht für große Mengen geeignet.

Hegna, I.K., Clausen O.G. 1988, An investigation of the bactericidal and fungicidal effects of certain disinfectants by use of capacity test. Ann. Inst. Pasteur., Microbiol. 139: 473 - 483; LeChevallier, M.W., and McFeters G.A. 1985b. Ennumerating injured coliforms in drinking water, J.Am. Water Works Assoc. 77: 81 - 87; Conn, H., Langer R. 1981, lodine disinfection of hydrophilic contact lenses. Ann. Ophthalmol. 13: 361 - 364 beschreiben anderweitige Zusätze von Chemikalien und Metallen. Auch diese Verfahren sind durch ständigen Substanzverbrauch, durch aufwendige Rückgewinnung der Substanzen, durch eine Gesundheitsschädigung durch die zugesetzten Substanzen, beispielsweise Formaldehyd, Phenol und Glutaraldehyd gekennzeichnet. In vielen Fällen eine Gesundheitsschädigung durch entstehende Nebenprodukte nicht auszuschließen.

Archer, A., Fischer E., Zellingher R., Manor Y. 1990, Photochemical disinfection of effluents-pilot plant studies. Wat. Res. 24: 837 - 843; Dahl, T.A., Midden W. R., Neckers D.C. 1988; Comparison of photodynamic action by Rose Bengal in gram-positive and gram-negative bacteria. Photochem. Photobiol. Vol. 48 No. 5: 607 - 612; Archer, A. J., Juven B. J. 1976, Destruction of Coliforms in Water and Sewage Water by Dye-Sensitized Photooxidation Appl. Environ. Microbiol. 33: 1019 - 1022 beschreiben die photodynamische Desinfektion mit in Wasser gelöstem Farbstoffen. Auch hier ist ein ständiger Substanzverbrauch erforderlich sowie eine Schwierigkeit der Rückgewinnung der Substanzen, beispielsweise Methylenblau oder Bengal Rosa, zu verzeichnen.

Die Filtration, die beispielsweise von Dorau, W. 1996, Mikrofiltration als Schlussreinigung bei der Abwasserreinigung, Notwendigkeit, verfahrenstechnische Einbindung, Kosten, Gewässerschutz-Wasser-Abwasser 152: 51/1-28 und Kolega, M., Grohmann G.S., Chiew R.F., Day A.W. 1991, Disinfection and clarification of treated sewage by advanced microfiltration. Water Sci. Technol. 23: 1609 - 1618 beschrieben wird, ist eine aufwendige Maßnahme zur Behandlung von Wasser. Die Filter verstopfen sehr leicht, so daß ein hoher Wartungsaufwand erforderlich ist, der eine regelmäßige mechanische und chemische Reinigung der Anlage bedingt.

**Im Zusammenhang mit photodynamischer Therapie beschreiben M. Hu et al. J. Med. Chem. 1998, 41, 1789-1802, hydroxysubstituierte Phthalocyanine. Diese Phthalocyanine weisen keine Sulfogruppe auf.**

Die bekannten photosensitiven Polymere auf Basis von Polymethylmethacrylat und Phthalocyaninen sind bisher entweder wasserlöslich oder unlöslich. Ein Polymer mit hydrophilem Charakter, das jedoch wasserunlöslich ist, ist nicht bekannt.

Die vorgenannte Aufgabe der vorliegenden Erfindung wird in einer ersten Ausführungsform gelöst durch metallierte oder unmetallierte Phthalocyanine der allgemeinen Formel I 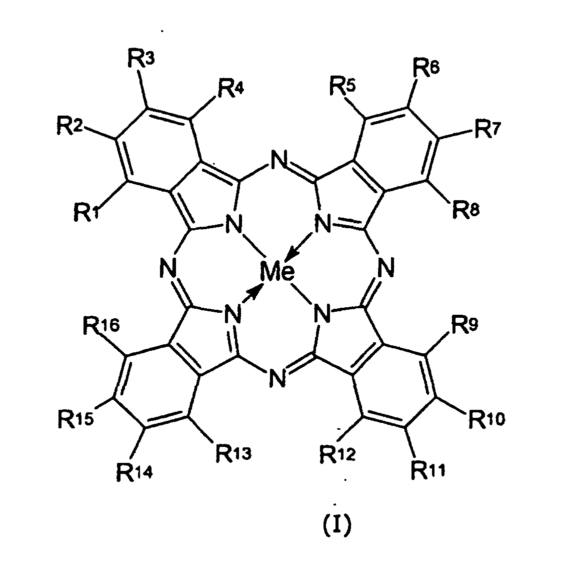 wobei
R¹ bis R¹⁶ gegebenenfalls für einen Teil eines gegebenenfalls N-enthaltenden benzokondensierten Rings mit Substituenten R^{1'} bis R^{16'} steht, R¹ bis R¹⁶, entsprechend R^{1'} bis R^{16'}, jeweils für Hydroxy oder einen C-O-C-verknüpften Polymerrest, wobei der Polymerrest abgeleitet ist von Polyestern, Polymethylmethacrylaten, Polymethacrylsäuren, Polyacrylsäuren und deren Estern, Polymethacrylamiden, Polyacetalen, Polyimiden und/oder Polyamiden sowie deren jeweilige Monomerbausteine umfasst, sowie für Wasserstoff, Alkyl, Aryl, Aralkyl oder Alkaryl, Alkyl-, Aryl-, Aralkyl- oder Alkarylsulfonat, sowie für SO₃H, F, Cl, Pyridin-N-Alkyl, -Aryl, -Alkaryl oder -Aralkyl mit jeweils 1 bis 8 C-Atomen mit den Maßgaben steht, dass genau einer der Reste R¹ bis R¹⁶ oder R^{1'} bis R^{16'} für eine Hydroxygruppe oder einen C-O-C-verknüpften Polymerrest steht,
wenigstens einer der Reste R¹ bis R¹⁶ oder R^{1'} bis R^{16'} für eine SO₃H-Gruppe steht, wobei
Me für ein Metallkation oder die Sättigung mit Wasserstoffatomen steht.

In einer zweiten Ausführungsform wird die vorgenannte Aufgabe gelöst durch metallierte oder unmetallierte Phthalocyanine der allgemeinen Formel II, 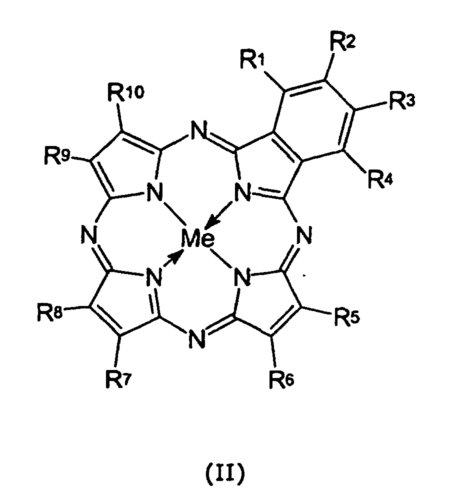 wobei
R¹ bis R¹⁰ gegebenenfalls für einen Teil eines gegebenenfalls N-enthaltenden benzokondensierten Rings mit Substituenten R^{1'} bis R^{10'} steht, R¹ bis R¹⁰, entsprechend R^{1'} bis R^{10'}, jeweils für Hydroxy oder einen C-O-C-verknüpften Polymerrest, wobei der Polymerrest abgeleitet ist von Polyestern, Polymethylmethacrylaten, Polymethacrylsäuren, Polyacrylsäuren und deren Estern, Polymethacrylamiden, Polyacetalen, Polyimiden und/oder Polyamiden sowie deren jeweilige Monomerbausteine umfasst sowie für Wasserstoff, Alkyl, Aryl, Aralkyl oder Alkaryl, Alkyl-, Aryl-, Aralkyl- oder Alkarylsulfonat sowie für SO₃H, F, Cl, Pyridin-N-Alkyl, -Aryl, -Alkaryl oder -Aralkyl mit jeweils 1 bis 8 C-Atomen mit den Maßgaben steht, dass genau einer der Reste R¹ bis R⁴ für eine Hydroxygruppe oder einen C-O-C-verknüpften Polymerrest steht, wenigstens eines der Gruppenpaare R⁵,/R⁶, R⁷/R⁸ und R⁹/R¹⁰ ein N-enthaltender benzokondensierter Ring mit entsprechenden Substituenten R^{5'} bis R^{10'} steht mit der Maßgabe, dass der Stickstoff alkyliert, aryliert, alkaryliert oder aralkyliert mit jeweils 1 bis 8 C-Atomen ist, wenigstens einer der Reste R¹ bis R¹⁰ oder R^{1'} bis R^{10'} für eine SO₃H-Gruppe steht, wobei
Me für ein Metallkation oder die Sättigung mit Wasserstoff steht.

Me steht insbesondere für ein Metallkation mit halbbesetzter oder vollbesetzter d-Elektronenschale, insbesondere Zink und Aluminium.

Die Formeln I und insbesondere II stellen vereinfachte Darstellungen der Phthalocyanine dar, die bedingt durch Substituentenvariation in verschiedensten Isomeren vorliegen können, die der Fachmann aufgrund der Umsetzung der Ausgangsstoffe erwarten kann. Hierunter fallen insbesondere Verbindungen, die unter gleichzeitiger Verwendung von Phthalsäure und Pyridindicarbonsäure hergestellt werden.

Die erfindungsgemäßen Phthalocyanine besitzen nur eine funktionelle Gruppe (HO-), die als Verknüpfungsstelle zum Polymer beziehungsweise Monomer verwendet wird. Daher wird im Polymer der Farbstoff nicht im Inneren der Matrix eingeschlossen, so daß der Sensibilisator frei verfügbar ist für die Anregung des Sauerstoffs zum Singulettsauerstoff. Farbstoffe mit mehr als einer Hydroxygruppe werden in das Polymer eingebaut, so daß sie keinen Kontakt mit der Umgebung bekommen und daher inaktiv oder nur gering aktiv sind.

Die Verknüpfungsstelle zwischen Polymer und Farbstoff wird durch eine C-O-C-Verknüpfung ausgebildet. Diese ist stabiler als eine vergleichbare Amidgruppe oder die Bindung über Ionenanziehung.

Die polaren Gruppen (SO₃H beziehungsweise N⁺-R) bewirken eine gute Benetzung des Polymers beim Kontakt mit Wasser, so daß hierdurch der Kontakt zum Wasser wesentlich verbessert wird, was für die Bildung des Singulettsauerstoffes essentiell ist. Die erfindungsgemäßen Phthalocyanine sind gegen Bestrahlung Langzeit-stabiler als Porphyrine.

Überraschenderweise wurde gefunden, daß die erstmals zur Verfügung gestellten, gegebenenfalls hydrophilen Phthalocyanine der allgemeinen Formel I und II effiziente Sensibilisatoren sind, die sich außerdem noch leicht kovalent an Polymere binden lassen oder gebunden sind. Durch die Modifikation der Substitutenten mit polaren Gruppen läßt sich die Hydrophilie dieser Polymere in einem breiten Bereich steuern, so daß auf der einen Seite hydrophobe Polymere (d.h. keine polaren Gruppen) und auf der anderen Seite sogar wasserlösliche Polymere (hoher Anteil polarer Gruppen) herstellbar sind. Zwischen diesen beiden Extremen stehen hydrophile, aber wasserunlösliche Polymere. Außerdem kann die Wasserlöslichkeit durch den Polymerisationsgrad des Basispolymers gesteuert werden.

In einer besonders bevorzugten Ausführungsform der vorliegenden Erfindung sind die metallierten oder unmetallierten Phthalocyanine der allgemeinen Formel I dadurch gekennzeichnet, daß R¹ bis R¹⁶ oder R^{1'} bis R^{10'} für Wasser-stoff mit der Maßgabe steht, daß **genau** einer der Reste R¹ bis R¹⁶ oder R^{1'} bis R^{10'} für eine Hydroxygruppe steht. Diese Verbindungen dienen vorzugs-weise als Ausgangsmaterial für die Umsetzung mit Monomerbausteinen oder Polymerbausteinen zur kovalenten Fixierung der Phthalocyanine, insbeson-dere durch Umesterung vorhandener Estergruppen.

Ein weiterer Aspekt der Erfindung ist die Einführung von Sulfonsäuregruppen in die Alkyl-, Aryl-, Alaryl- oder Aralkylgruppen des Phthalocyaninderivats. Dabei kann der Sulfonierungsgrad über die Zugabe des Sulfonierungsmittel gezielt gesteuert werden, wodurch der hydrophile Charakter des Polymers geändert werden kann. Die Sulfonierung kann vor der Herstellung der Monomeren durchgeführt werden oder nach der Darstellung der Polymeren.

Ein weiterer Aspekt der Erfindung ist die Einführung von Metallen (Me), insbesondere mit halbgefüllter d-Elektonenschale, nachdem das unmetallierte photosensitive Polymer vorliegt. Besonders bevorzugt enthalten die Phthalocyaninderivate Zink als zentrales Kation.

Ein besonderer Aspekt der Erfindung ist die Darstellung von phthalocyaninhaltigen Polymethylmethacrylaten über die Umesterung der vorhandenen Estergruppen. Dabei können die Phthalocyanine metalliert oder unmetalliert sein.

Eine weitere bevorzugte Ausführungsform der vorliegenden Erfindung besteht in einem Verfahren zur Herstellung von Phthalocyaninen der allgemeinen Formel I, enthaltend einen C-O-C-gebundenen Monomerbaustein eines Polymeresters, durch Umsetzung von gegebenenfalls substituierter Phthalsäure, (3- und/oder 4-)Hydroxyphthalsäure und Harnstoff gefolgt durch Polymerisation oder durch Umsetzung von 2,3-Naphthalindicarbonsäure, Hydroxyphthalsäure und Harnstoff, gefolgt durch Polymerisation.

Alternativ können die Phthalocyaninderivate der allgemeinen Formel II, enthaltend einen C-O-C-gebundenen Monomerbaustein eines Polymeresters, durch Umsetzung von gegebenenfalls substituierter Pyridin-dicarbonsäure (2,3- oder 3,4), (3- und/oder 4-)Hydroxyphthalsäure und Harnstoff gefolgt durch Polymerisation erhalten werden.

Der Begriff Polymerisation beinhaltet im Sinne der vorliegenden Erfindung die Umsetzung mit Monomeren ebenso wie die Umsetzung mit Polymeren.

Besonders bevorzugt im Sinne der vorliegenden Erfindung werden die obengenannten Phthalocyanine mit Polymethylmethacrylat und/oder Polycarbonaten umgesetzt.

Besonders bevorzugt im Sinne der vorliegenden Erfindung wird das Phthalocyaninderivat der allgemeinen Formel I oder 11 mit einem Sulfonierungsmittel, insbesondere Chlorsulfonsäure oder konzentrierter Schwefelsäure nach an sich bekannten Verfahren umgesetzt.

Die mit Hilfe der vorliegenden Erfindung erhaltenen Photosensibilisatoren sind über eine kovalente Bindung an das Polymergerüst angebunden und ragen aus dem Polymer heraus. Sie sind keine im Inneren des Polymer eingebetteten Knotenpunkte.

Eine weitere Ausführungsform der vorliegenden Erfindung betrifft Photosensibilisatoren, die wenigstens ein Phthalocyaninderivat der allgemeinen Formel I und/oder II in einer Menge von 0,1 bis 10 Gew.-% enthalten. Hierbei liegt das Phthalocyaninderivat der allgemeinen Formel I und/oder II kovalent oder in nicht kovalent gebundener Form in einer Polymermatrix vor. Besonders bevorzugt im Sinne der vorliegenden Erfindung ist die kovalente Fixierung der Phthalocyanine in dem Trägermaterial.

Als polymere Trägermaterialien sind vor allem PMMA und Polycarbonate für technische Anwendungen interessant, da sie vorteilhafte Eigenschaften aufweisen: preiswert, mechanisch stabil, witterungsbeständig, lichtbeständig, transparent im Bereich der zur Anregung der Sensibilisatoren erforderlichen Lichtes.

Die Polymermatrix kann erfindungsgemäß bevorzugt aus Fasern, Granulaten, Vliesen, Geweben, Gewirken, Gestricken oder auch beliebigen Formkörpern, beispielsweise Membranen bestehen.

Ein weiterer Aspekt der Erfindung ist die Verwendung der photosensitiven Monomeren und Polymeren als Sensibilisatoren für photochemische Reaktionen. Die dargestellten Monomere und Polymere eignen sich für die Übertragung von aus Licht aufgenommener Energie auf chemische Substanzen.

Für Anwendungen, bei denen eine Langzeitstabilität der polymerfixierten Sensibilisatoren in wäßriger Umgebung gefordert wird, ist es von Nachteil, die Phthalocyanine an Cellulose zu binden, da Cellulose langsam hydrolysiert. Dieser Nachteil besteht nicht in dem Fall der kovalenten Bindung an PMMA.

Die Verwendung der insbesondere an PMMA fixierten Phthalocyanine in wäßrigen Systemen ruft nach Versuchen der vorliegenden Erfindung Singulett-Sauerstoff-Bildung hervor. Die erfindungsgemäß hergestellten Farbstoffe besitzen eine hohe Hydrophilie und sind somit unter anderem in der Lage, gute Singulett-Sauerstoff-Bildungsraten zu erzeugen.

Das erfindungsgemäß bevorzugte Verfahren zur Herstellung der Phthalocyanine über die Veresterung liefert ein Produkt innerhalb weniger Stunden.

Eine weitere Aufgabe der vorliegenden Erfindung besteht in einer Verbesserung der Behandlung von insbesondere Wasser oder Wasser enthaltenden Umgebungen durch Bestrahlung mit aktinischem, d. h. sichtbarem Licht. Weiterhin besteht die Aufgabe der vorliegenden Erfindung in der Bereitstellung von Gegenständen oder Behältern zur Desinfektion und/oder Detoxifikation von Gegenständen in Sauerstoff und insbesondere Wasser und/oder Wasser enthaltenen Umgebungen.

Die vorgenannte Aufgabe wird in einer Ausführungsform gelöst durch die Verwendung der obengenannten Phthalocyanine zur Detoxifizierung und/oder Desinfektion von Sauerstoff und insbesondere Wasser oder Wasser enthaltenen Umgebungen und durch Bestrahlung mit aktinischem Licht.

Die besonderen Vorteile der vorliegenden Erfindung bestehen in einem geringen Energieaufwand zum Anlagenbetrieb. Es kann in einem Arbeitsschritt desinfiziert und dekontaminiert werden. Durch die Bindung an ein polymeres Trägermaterial ist eine Rückgewinnungsproblematik bei der vorliegenden Erfindung nicht gegeben. Die polymergebundenen Phthalocyanine verbleiben in dem System, so daß keine permanenten Kosten durch ständigen Zusatz von Verbindungen erforderlich sind. Es werden keine toxischen Nebenprodukte oder Farbstoffe gebildet, - auch treten keine Abbauprodukte der metallierten und/oder unmetallierten polymergebundenen Phthalocyanine auf. Da die Substanz nicht toxisch ist, sind keine zusätzlichen Sicherheitsmaßnahmen erforderlich. Das erfindungsgemäße Verfahren der Verwendung von metallierten und/oder unmetallierten polymergebundenen Phthalocyaninen ist somit auch für die Reinigung großer Volumina geeignet. In sonnenreichen Gegenden sind keine zusätzlichen Lichtquellen notwendig. In Kombination mit kleinen mobilen Einheiten kann das erfindungsgemäße Prinzip auch in entlegenen Gegenden eingesetzt werden.

Es war überraschend, daß die polymergebundenen Phthalocyanine bei Kontakt mit Wasser oder Wasser enthaltenden Umgebungen quellen und somit auch hier in vorteilhafter Weise Singulett-Sauerstoff bei der Bestrahlung mit aktinischem (sichtbarem) Licht bilden.

Besonders bevorzugt im Sinne der vorliegenden Erfindung werden die Phthalocyanine zur Behandlung von Wasser oder Wasser enthaltenen Umgebungen in Kläranlagen eingesetzt. Zur Nachreinigung von kontaminiertem Wasser kann dieses derart aufbereitet werden, daß das Wasser als Brauchwasser innerhalb der Anlage rückgeführt werden kann. Diese grundsätzliche Rückführungsmöglichkeit besteht nicht nur bei kommunalen und industriellen Kläranlagen sondern auch beispielsweise für die Lebensmittelindustrie, wo Obst und Gemüse zur Konservenherstellung gewaschen werden. In der Textilindustrie, insbesondere in Gerbereien und überall dort, wo große Mengen Wasser anfallen, welches man auf diesem Wege dem hausinternen Brauchwasserkreislauf wieder rückführen und damit die Kosten für Wasser beziehungsweise Abwasser erheblich reduzieren kann, ist mit Hilfe der vorliegenden Erfindung eine Behandlung des Wassers möglich. Die vorliegende Erfindung eignet sich auch insbesondere für die Behandlung von Wasch-/Bade- und Duschwasser von großen Hotelanlagen oder Campingplätzen. Auch ist mit Hilfe der vorliegenden Erfindung eine permanente Desinfektion von Schwimmbädern möglich, wobei der Chlorverbrauch und somit die Umwelt und Gesundheitsbelastung sowie die Betriebskosten erheblich gesenkt werden können.

Im Weltraum ist beispielsweise die Aufbereitung des Brauchwassers in transparenten Tanks möglich.

Darüber hinaus ist das erfindungsgemäße Verfahren einsetzbar zur Abluftreinigung oder Umluftreinigung, insbesondere von Luft aus Viehställen oder zum Desodorieren von Abluft- und Umluftströmen in Gebäuden, beispielsweise Kaufhäusern.

In einer besonders bevorzugten Ausführungsform der vorliegenden Erfindung werden die oben genannten Phthalocyanine in Form von Flachbett-Reaktoren oder Blasensäulen-Reaktoren eingesetzt.

Eine weitere bevorzugte Ausführungsform der vorliegenden Erfindung besteht darin, daß man die oben genannten Phthalocyanine als ortsfeste Strukturen, insbesondere Platten aus Vollmaterial oder beschichtete Platten sowie als Granulat, Pulver, Membran oder Filter einsetzt.

Neben den oben genannten wäßrigen Systemen, die mit Hilfe der vorliegenden Erfindung detoxifiziert oder dekontaminiert werden können, ist es in einer weiteren Ausführungsform der vorliegenden Erfindung möglich, Gegenstände aller Art mit den obengenannten Phthalocyaninen zu desinfizieren und/oder zu detoxifizieren. Besonders bevorzugt im Sinne der vorliegenden Erfindung werden daher diese Gegenstände in eine wäßrige oder Wasser enthaltende Umgebung eingebracht und so mit den Phthalocyaninen in Kontakt gebracht.

Eine besondere Ausführungsform der vorliegenden Erfindung besteht somit darin, Behälter oder Gegenstände zur Detoxifizierung und/oder Desinfektion von Wasser oder Wasser enthaltenden Umgebungen bereitzustellen. Im Sinne der vorliegenden Erfindung umfassen die genannten Behälter oder Gegenstände Oberflächenbereiche aus polymergebunden Phthalocyaninen. Hierzu werden an sich bekannte Behälter oder Gegenstände hergestellt, die wenigstens in Teilbereichen der Oberfläche die polymergebundenen Phthalocyanine enthalten.

So können die oben beschriebenen Behälter beispielsweise Reinigungsgeräte für Kontaktlinsen, Zahnersatz oder Zahnregulatorien umfassen. Eine weitere Ausführungsform der vorliegenden Erfindung besteht somit aus Gegenständen, die antibakterielle Beschichtungen von Oberflächen in der Medizintechnik umfassen, wobei diese Oberflächen metallierte oder unmetallierte polymergebundene Phthalocyanine enthalten.

Die erfindungsgemäße Verwendung der Phthalocyanine für die Reinigung von Wasser oder Luft beruht auf der Produktion von Singulett-Sauerstoff.

Eine allgemeine Übersicht der Reaktionen, die mit Singulett-Sauerstoff durchgeführt werden können, ist in Houben Weyl, Methoden der organischen Chemie, Photochemie Band II, Georg Thieme Verlag Stuttgart, 1975, S. 1465 beschrieben. Als chemische Substanzklassen werden dort Alkene, Aromaten, Heteroaromaten, Stickstoff-, Schwefel-, Phosphorverbindungen und andere aufgeführt. Diese können in ein höher oxidiertes Derivat überführt werden, die so für eine biologische Klärung des Wassers vorbereitet werden. Die höher oxidierten Stoffe im Falle der Schwefelverbindungen besitzen einen reduzierten Eigenduft, so daß hier eine desodorierende Wirkung zu beobachten ist.

Daraus läßt sich prinzipiell ableiten, daß Singulett-Sauerstoff produzierender Farbstoff grundsätzlich in Wasser als Sensibilisator für Wasserreinigung eingesetzt werden kann.

Die Reaktivität von Singulett-Sauerstoff in der Gasphase ist in verschiedenen Pulbikationen nachgewiesen worden, wie zum Beispiel in Eisenberg, Walter C.; DeSilva, Mutha, Journal, TELEAY, Tetrahedron Lett., EN, 31,41, 1990, 5857-5860, "Atmospheric gas phase generation of singlet oxygen by homogeneous photosensitization"; Borisov, A.V., Tsivenko, V.I., Myasinkov, I.A., Venediktor E.A., Journal RJPACAR, Russ. J. Phys. Chem. (Engl. Transl.) EN, 64,6,1990, 922 - 924, "Photostimulated formation and emission of singlet oxygen from the surface of metallo-tetraphenylporphins adsorbed on silica" und Borisov, A.V.; Tsivenko, V.I., Myasnikov, I.A. Journal, RJPCAR, Russ. J. Phys. Chem. (Engl. Transl.), EN, 66, 2, 1992, 307 - 308, "Photostimulated emission of singlet oxygen molecules into the gas phase from tetra(m-butoxyphenyl)porphin adsorbed on SiO₂".

Zur Behandlung von Abluft und Abgasen sind folgende Verfahren bekannt:
1. Abscheidung gasförmiger Stoffe durch Absorption, Kondensation, Membran-Permeation und Trockensorption
   Der Nachteil besteht in dem Verbrauch von chemischen Hilfsreagenzien, zum Beispiel Aktiv-Kohle, Kalk etc.
2. Abbau von Dioxinen und Furanen in Abgasen mit Wasserstoffperoxid Der Nachteil besteht in dem Verbrauch an Wasserstoffperoxid
3. Abscheidung gasförmiger Schadstoffe durch katalytische Reaktionen wie Oxidation, Reduktion, und Zersetzungsreaktionen. Edelmetallkatalysatoren, Schwermetallkatalysatoren und oxidische Katalysatoren kommen zum Einsatz. Der Nachteil besteht in dem Abrieb, wobei eine Vergiftung der Katalysatoren möglich ist. Teilweise sind die Katalysatoren nur bei erhöhten Temperaturen wirksam.
   Bei den Oxidationskatalysatoren wird meist teureres Platinmetall verwendet. Teures Ozon wird für die Entkeimung von Abluft eingesetzt. Der Überschuß muß wieder zerstört werden.
4. Abscheidung gasförmiger Schadstoffe durch biologische Reaktionen: Biowäscher (Absorption in einer Flüssigkeit, die mit Mikroorganismen versetzt ist), Biofilter (sowohl Adsorption an einer Feststoffoberfläche als auch Absorption in einer Flüssigkeit) und Biomembranfilterverfahren.

Die Verfahren sind dem Handbuch des Umweltschutzes und der Umweltschutztechnik, Band 3: Additiver Umweltschutz: Behandlung von Abluft und Abgasen, von H. Bauer (Hrsg.), Springer Verlag, Berlin 1996 entnommen.

Alle Verfahren, die zum Beispiel zur Herstellung von Gegenständen aus PMMA angewendet werden, können auch erfindungsgemäß verwendet werden, sofern die Temperaturen unter 200 °C liegen.

Eine weitere Ausführungsform der Erfindung umfaßt daher die Verwendung von Photosensibilisatoren gemäß der vorliegenden Erfindung zur Bildung von Singulett-Sauerstoff aus Triplett-Sauerstoff durch Einwirkung von sichtbarem Licht, insbesondere zur Reinigung von kontaminierten Flüssigkeiten, beispielsweise Wasser.

Eine besondere Ausführungsform der Erfindung besteht darin, daß Metallierung, Sulfonierung, Alkylierung erst am fertigen modifizierten Polymer erfolgen kann. Hierdurch ergibt sich insbesondere die Möglichkeit zur gezielteren Steuerung der jeweiligen Grade.

### Ausführungsbeispiele:

### Beispiel 1

### Darstellung von Monohydroxyphthalocyanin

3 mol Phthalsäure, 1 mol Monohydroxyphthalsäure und 4 mol Harnstoff werden gemischt und bei 160 °C 30 min lang erhitzt. Der sich bildende Farbstoff wird nach den gängigen Methoden gereinigt, wie Waschen mit Säure und Laugen.

### Beispiel 2

### Darstellung von Zinkmonohydroxyphthalocyanin

3 mol Phthalsäure, 1 mol Monohydroxyphthalsäure und 4 mol Harnstoff werden in Gegenwart von 1 mol Zinkchlorid gemischt und bei 160 °C 30 min lang erhitzt. Der sich bildende Farbstoff wird nach den gängigen Methoden gereinigt, wie Waschen mit Säure und Laugen.

### Beispiel 3

### Darstellung von persulfoniertem Zinkmonohydroxyphthalocyanin

Eine Mischung aus 1 mol Zinkmonohydroxyphthalocyanin in Chloroform wird bei 0 °C mit 6 mol Chlorsulfonsäure gelöst in 10 ml Chloroform versetzt. Nach 12 h wird die Lösung auf Eis gegossen und die organische Phase separiert. Nach dem Einengen der wässrigen Phase erhält man das Produkt.

### Beispiel 4

### Darstellung von Zink(II)-monohydroxytrispyridiniumphthalocyanin

3 mol Pyridindicarbonsäure, 1 mol Monohydroxyphthalsäure und 4 mol Harnstoff werden in Gegenwart von 1 mol Zinkchlorid gemischt und auf 160 °C 30 min lang erhitzt. Der sich bildende Farbstoff wird nach den gängigen Methoden gereinigt, wie Waschen mit Säure und Laugen.

### Beispiel 5

### Darstellung von Zink(II)-methacryloyloxyphenyltripyridiniumphthalocyanin

In 20 ml DMF wird 1 mol Zink(II)-monohydroxytrispyridiniumphthalocyanin gelöst und mit 1 mol Methacrylsäure in Gegenwart von 2 mol Triethylamin versetzt. Nach dem Abreagieren wird das Lösungsmittel entfernt und das photosensitive Monomer als Rückstand gewonnen.

### Beispiel 6

### Darstellung von fixiertem Zink(II)monohydroxytrispyridiniumphthalocyanin

Eine Mischung aus Polymethylmethacrylat und Zink(II)-monohydroxytrispyridiniumphthalocyanin in DMF wird in Gegenwart von p-Toluolsulfonsäure erwärmt. Dabei wird das Polymer umgeestert. Durch mehrfaches Abdestillieren des DMF und erneuter Aufnahme des Rückstands in DMF wird der Farbstoff vollständig fixiert.

Zink(II)-methacyloyloxphenyltrispyridiniumphthalocyanin wird mit Methacrylsäuremethylester in DMF gelöst. Die Polymerisation wird mit AIBN gestartet. Das Entfernen des Lösungsmittels liefert das gewünschte Polymer als dunkelblaues Pulver.

### Beispiel 7

### Methylierung des fixierten Zink(II)-monohydroxytrispyridiniumphthalocyanin

1 mol Zink(II)-monohydroxytrispyridiniumphthalocyanin wird mit 3 mol Methyl-p-toluolsulfonat in DMF bei 160°C erhitzt. Nach dem Entfernen des Lösungsmittels erhält man das tris-methylierte Produkt.

### Beispiel 8

### Bestimmung der Singulett-Sauerstoff-Produktionsrate von fixiertem, methylierten Zink(II)-monohydroxytrispyridiniumphthalocyanin

Die Befähigung hydrophiler Farbstoffe, Singulett-Sauerstoff zu produzieren, wird über einen einfachen Test geprüft. Die Produktionsraten werden durch die Bleichung von p-Nitrosodimethylamilin (RNO) bestimmt.

10 ml einer RNO-Lösung werden mit dem Farbstoff (2 ppm) versetzt und bestrahlt. Als Referenzgröße wird der Wert des Bengalrosas von 2,6 10⁻⁸ mol/l/s verwendet.

Für fixiertes, methyliertes Zink(II)-monohydroxytrispyridiniumphthalocyanin wurde ein Wert von 3,7 10⁻⁹ mol/l/s bestimmt.

Das persulfonierte Kupfer(II)-Phthalocyanin liefert einen Wert von 2 10⁻⁹ mol/l/s.

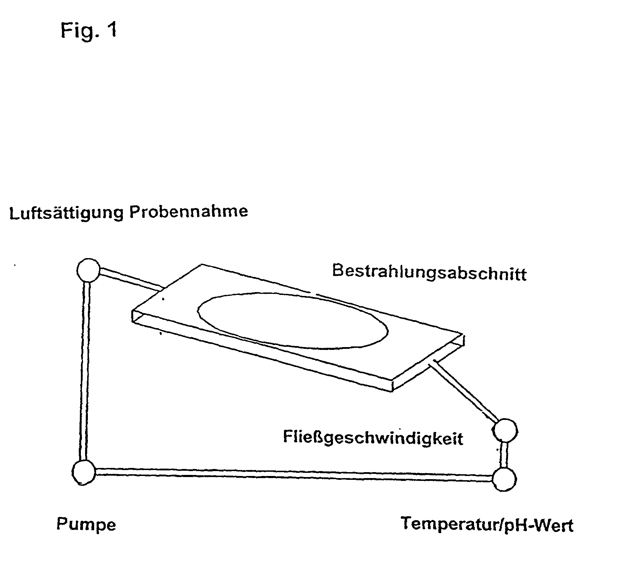

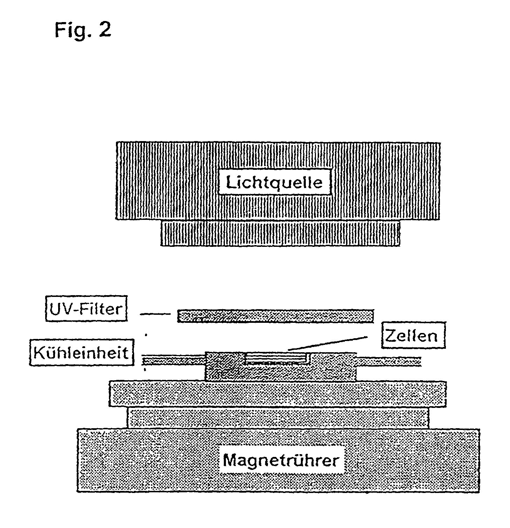

## Patentansprüche

1. Metallierte oder unmetallierte Phthalocyanine der allgemeinen Formel I 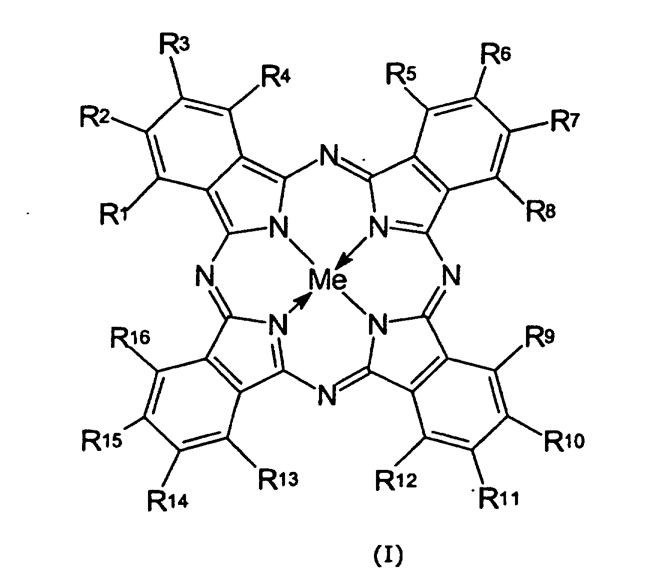 wobei
R¹ bis R¹⁶ gegebenenfalls für einen Teil eines gegebenenfalls N- enthaltenden benzokondensierten Rings mit Substituenten R^{1'} bis R^{16'} steht, R¹ bis R¹⁶, entsprechend R^{1'} bis R^{16'}, jeweils für Hydroxy oder einen C-O-C-verknüpften Polymerrest, wobei der Polymerrest abgeleitet ist von Polyestern, Polymethylmethacrylaten, Polymethacrylsäuren, Polyacrylsäuren und deren Estern, Polymethacrylamiden, Polyacetalen, Polyimiden und/oder Polyamiden sowie deren jeweilige Monomerbausteine umfasst, sowie für Wasserstoff, Alkyl, Aryl, Aralkyl oder Alkaryl, Alkyl-, Aryl-, Aralkyl- oder Alkarylsulfonat, sowie für SO₃H, F, Cl, Pyridin-N-Alkyl, -Aryl, -Alkaryl oder -Aralkyl mit jeweils 1 bis 8 C-Atomen mit den Maßgaben steht, dass genau einer der Reste R¹ bis R¹⁶ oder R^{1'} bis R^{16'} für eine Hydroxygruppe oder einen C-O-C-verknüpften Polymerrest steht,
wenigstens einer der Reste R¹ bis R¹⁶ oder R^{1'} bis R^{16'} für eine SO₃H-Gruppe steht,
wobei
Me für ein Metallkation oder die Sättigung mit Wasserstoffatomen steht.

2. Metallierte oder unmetallierte Phthalocyanine der allgemeinen Formel II, 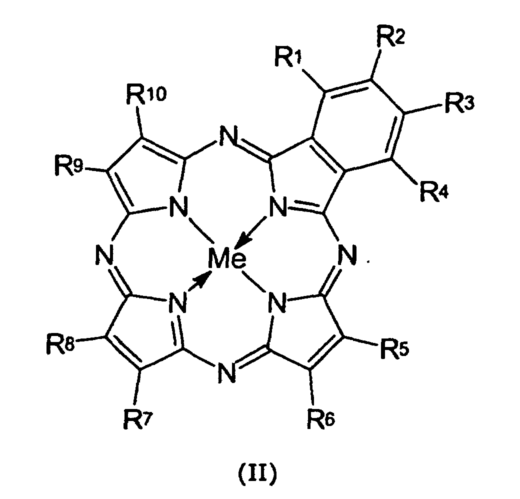 wobei
R¹ bis R¹⁰ gegebenenfalls für einen Teil eines gegebenenfalls N-enthaltenden benzokondensierten Rings mit Substituenten R^{1'} bis R^{10'} steht, R¹ bis R¹⁰, entsprechend R^{1'} bis R^{10'}, jeweils für Hydroxy oder einen C-O-C-verknüpften Polymerrest, wobei der Polymerrest abgeleitet ist von Polyestern, Polymethylmethacrylaten, Polymeth-acrylsäuren, Polyacrylsäuren und deren Estern, Polymethacrylamiden, Polyacetalen, Polyimiden und/oder Polyamiden sowie deren jeweilige Monomerbausteine umfasst sowie für Wasserstoff, Alkyl, Aryl, Aralkyl oder Alkaryl, Alkyl-, Aryl-, Aralkyl- oder Alkarylsulfonat sowie für SO₃H, F, Cl, Pyridin-N-Alkyl, -Aryl, -Alkaryl oder -Aralkyl mit jeweils 1 bis 8 C-Atomen mit den Maßgaben steht, dass genau einer der Reste R¹ bis R⁴ für eine Hydroxygruppe oder einen C-O-C-verknüpften Polymerrest steht, wenigstens eines der Gruppenpaare R⁵,/R⁶, R⁷/R⁸ und R⁹/R¹⁰ ein N-enthaltender benzokondensierter Ring mit entsprechenden Substituenten R^{5'} bis R^{10'} steht mit der Maßgabe, dass der Stickstoff alkyliert, aryliert, alkaryliert oder aralkyliert mit jeweils 1 bis 8 C-Atomen ist, wenigstens einer der Reste R¹ bis R¹⁰ oder R^{1'} bis R^{10'} für eine SO₃H-Gruppe steht,
wobei
Me für ein Metallkation oder die Sättigung mit Wasserstoff steht.

3. Metallierte oder unmetallierte Phthalocyanine der allgemeinen Formel I oder II, wobei R¹ bis R¹⁶, R¹ bis R¹⁰ für Wasserstoff mit der Maßgabe steht, dass genau einer der Reste R¹ bis R¹⁶ oder R¹ bis R⁴ für eine Hydroxygruppe steht.

4. Metallierte Phthalocyaninderivaten nach Anspruch 1 und/oder 2, wobei Me für Zn oder Al steht.

5. Verfahren zur Herstellung von Phthalocyaninderivaten der allgemeinen Formel I und/oder II gemäß Anspruch 1 und/oder 2, enthaltend einen C-O-C-gebundenen Monomerbaustein eines Polymerrestes, durch Umsetzung von gegebenenfalls substituierter Phthalsäure, Hydroxyphthalsäure und Harnstoff, gefolgt durch Polymerisation.

6. Verfahren zur Herstellung von Phthalocyaninderivaten der allgemeinen Formel I und II gemäß Anspruch 1 und/oder 2, enthaltend einen C-O-C-gebundenen Monomerbaustein eines Polymerrestes, durch Umsetzung von 2,3-Naphthalindicarbonsäuren, Hydroxyphthalsäure und Harnstoff, gefolgt durch Polymerisation.

7. Verfahren zur Herstellung von Phthalocyaninderivaten der allgemeinen Formel I und/oder II gemäß Anspruch 1 und/oder 2, enthaltend einen C-O-C-gebundenen Monomerbaustein eines Polymerrestes, durch Umsetzung von gegebenenfalls substituierter Pyridindicarbonsäure, Hydroxyphthalsäure und Harnstoff, gefolgt durch Polymerisation.

8. Verfahren nach Anspruch 5 bis 7, **dadurch gekennzeichnet, dass** man Phthalocyaninderivat der allgemeinen Formel I oder II mit einem Sulfonierungsmittel, insbesondere Chlorsulfonsäure oder konzentrierter Schwefelsäure umsetzt.

9. Verfahren zur Herstellung von Phthalocyaninderivaten der allgemeinen Formel I gemäß Anspruch 1 und/oder **der allgemeinen Formel II gemäß Anspruch 2** durch Umsetzung von **Hydroxyphthalsäure und Harnstoff mit ge**gebenenfalls substituierter Phthalsäure, **insbesondere 2,3-Naphthalindicarbonsäure, dadurch gekennzeichnet, dass man die erhaltenen hydroxygruppenhaltigen** Phthalocyaninderivate mit Polymethylmethacrylat oder Polycarbonat umsetzt.

10. Photosensibilisator umfassend wenigstens ein Phthalocyaninderivat der allgemeinen Formel I gemäß Anspruch 1 und/oder **der allgemeinen Formel II gemäß Anspruch 2** in einer Menge von 0,1 bis 10 Gew.-%.

11. Photosensibilisator nach Anspruch 10, **dadurch gekennzeichnet, dass** das Phthalocyaninderivat der allgemeinen Formel I und/oder II kovalent gebunden mit einer Polymermatrix vorliegt.

12. Photosensibilisator nach Anspruch 11, **dadurch gekennzeichnet, dass** die Polymermatrix Fasern, Granulate, Pulver, Vliese, Gewebe, Gewirke, Gestrike oder Formkörper, insbesondere Membranen umfasst.

13. Verwendung von Photosensibilisatoren nach einem oder mehreren der Ansprüche 10 bis 12 zur Auslösung photochemischer Reaktionen durch Einwirkung von sichtbarem Licht.

14. Verwendung von Photosensibilisatoren nach Anspruch 13 zur katalytischen Bildung von Singulett-Sauerstoff aus Triplett-Sauerstoff durch Einwirkung von sichtbarem Licht.

15. Verwendung von metallierten und/oder unmetallierten polymergebundenen Phthalocyaninen nach Anspruch 13 zur Detoxifizierung und/oder Desinfektion von Sauerstoff und insbesondere Wasser oder Wasser enthaltenden Umgebungen durch Bestrahlung mit Licht, insbesondere aktinischem Licht.

16. Verwendung nach Anspruch 13 zur Aufbereitung von Brauchwasser, insbesondere in Wasserkreisläufen; als Zwischenreinigung oder Endreinigung in industriellen oder kommunalen Kläranlagen; zur Waschwasser-Reinigung in der Lebensmittelindustrie, insbesondere beim Waschen von Obst und Gemüse vor der Konservierung; zur Reinhaltung von Wasser-Speichern; zur Trinkwasserstabilisierung; in Kombination mit Gaswäschern, bei dem belastete Waschwässer anfallen; bei der Aufbereitung von Wasch-, Bade- und Duschwasser von industriellen und kommunalen Anlagen, insbesondere Hotelanlagen oder Campingplätzen; zur Reinigung von Schwimmbadwässern, insbesondere in Kombination mit solarer Wassererwärmung; zur Abluftreinigung oder Umluftreinigung, insbesondere von Luft aus Viehställen oder zum Desodorieren von Abluft- und Umluftströmen in Gebäuden, zur Desinfektion und/oder Detoxifikation von Gegenständen aller Art einsetzt.

17. Verwendung nach Anspruch 13, **dadurch gekennzeichnet, dass** man die Phthalocyanine als ortsfeste Strukturen, insbesondere Platten aus Vollmaterial oder beschichtete Platten sowie als Granulate, Pulver, Membrane oder Filter einsetzt.

18. Verwendung nach Anspruch 13, **dadurch gekennzeichnet, dass** man Phthalocyanine in Form von Flachbett-Reaktoren oder Blasensäulen-Reaktoren einsetzt.

19. Behälter oder Gegenstände zur Detoxifizierung und/oder Desinfektion von Gegenständen, Wasser oder Wasser enthaltenden Umgebungen umfassend Oberflächenbereiche aus polymergebundenen Phthalocyaninderivaten nach Anspruch 1.

20. Behälter nach Anspruch 19 umfassend Reinigungsgeräte für Kontaktlinsen, Zahnersatz oder Zahnregulatorien.

21. Gegenstände nach Anspruch 19, umfassend antibakterielle Beschichtungen von Oberflächen in der Medizintechnik.

## Claims

1. Metallated or unmetallated phthalocyanines of general formula I 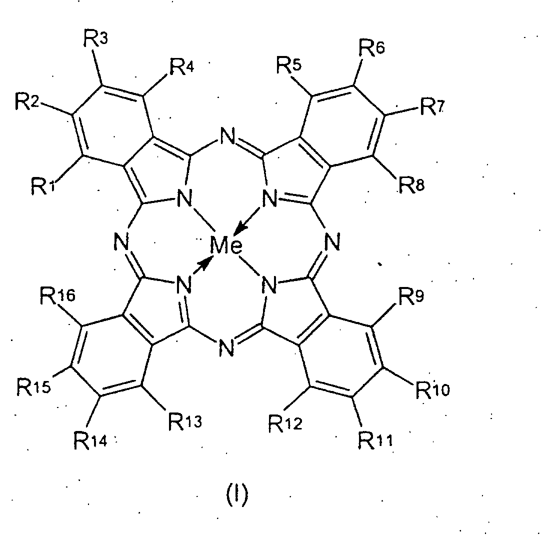 wherein
R¹ to R¹⁶ optionally represent part of an optionally N-containing benzo-anellated ring with substituents R^{1'} to R^{16'}, R¹ to R¹⁶, corresponding to R^{1'} to R^{16'}, respectively represent hydroxy or a C-O-C linked polymer residue, said polymer residue being derived from polyesters, polymethyl methacrylates, polymethacrylic acids, polyacrylic acids and their esters, polymethacrylamides, polyacetals, polyimides and/or polyamides, and their respective monomer units, and represent hydrogen, alkyl, aryl, aralkyl or alkaryl, alkyl-, aryl-, aralkyl- or alkarylsulfonate, and SO₃H, F, Cl, pyridine-N-alkyl, - aryl, -alkaryl or -aralkyl each having from 1 to 8 carbon atoms, with the proviso that only one of residues R¹ to R¹⁶ or R¹' to R¹⁶' represents a hydroxy group or a C-O-C linked polymer residue, at least one of residues R¹ to R¹⁶ or R^{1'} to R^{16'} represents an SO₃H group;
wherein Me represents a metal cation or saturation with hydrogen atoms.

2. Metallated or unmetallated phthalocyanines of general formula II 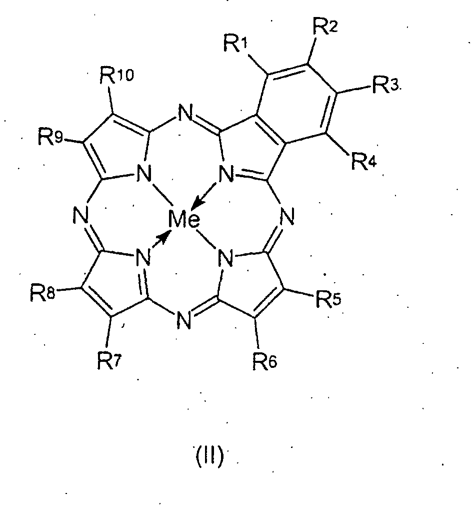 wherein
R¹ to R¹⁰ optionally represent part of an optionally N-containing benzo-anellated ring with substituents R^{1'} to R^{10'}, R¹ to R¹⁰, corresponding to R^{1'} to R^{10'}, respectively represent hydroxy or a C-O-C linked polymer residue, said polymer residue being derived from polyesters, polymethyl methacrylates, polymethacrylic acids, polyacrylic acids and their esters, polymethacrylamides, polyacetals, polyimides and/or polyamides, and their respective monomer units, and represent hydrogen, alkyl, aryl, aralkyl or alkaryl, alkyl-, aryl-, aralkyl- or alkarylsulfonate, and SO₃H, F, Cl, pyridine-N-alkyl, -aryl, -alkaryl or -aralkyl each having from 1 to 8 carbon atoms, with the proviso that only one of residues R¹ to R⁴ represents a hydroxy group or a C-O-C linked polymer residue, at least one of the pairs of groups R⁵/R⁶, R⁷/R⁸ and R⁹/R¹⁰ represents an N-containing benzo-anellated ring with corresponding substituents R^{5'} to R^{10'}, with the proviso that said nitrogen is alkylated, arylated, alkarylated or aralkylated, each with from 1 to 8 carbon atoms, and at least one of residues R¹ to R¹⁰ or R^{1'} to R^{10'} is an SO₃H group; wherein Me represents a metal cation or saturation with hydrogen.

3. Metallated or unmetallated phthalocyanines of general formula I or II wherein R¹ to R¹⁶, R¹ to R¹⁰ represent hydrogen, with the proviso that only one of residues R¹ to R¹⁶ or R¹ to R⁴ represents a hydroxy group.

4. Metallated phthalocyanine derivatives according to claims 1 and/or 2 wherein Me represents Zn or Al.

5. A process for the preparation of phthalocyanine derivatives of general formulae I and/or II according to claims 1 and/or 2 containing a C-O-C linked monomer unit of a polymer residue by reacting optionally substituted phthalic acid, hydroxyphthalic acid and urea, followed by polymerization.

6. A process for the preparation of phthalocyanine derivatives of general formulae I and II according to claims 1 and/or 2 containing a C-O-C linked monomer unit of a polymer residue by reacting 2,3-naphthalenedicarboxylic acids, hydroxyphthalic acid and urea, followed by polymerization.

7. A process for the preparation of phthalocyanine derivatives of general formulae I and/or II according to claims 1 and/or 2 containing a C-O-C linked monomer unit of a polymer residue by reacting optionally substituted pyridinedicarboxylic acid, hydroxyphthalic acid and urea, followed by polymerization.

8. The process according to claims 5 to 7, **characterized in that** said phthalocyanine derivative of general formula I or II is reacted with a sulfonating agent, especially chlorosulfonic acid or concentrated sulfuric acid.

9. A process for the preparation of phthalocyanine derivatives of general formula I according to claim 1 and/or of general formula II according to claim 2 by reacting hydroxyphthalic acid and urea with optionally substituted phthalic acid, especially 2,3-naphthalenedicarboxylic acid, **characterized in that** the hydroxy group containing phthalocyanine derivatives obtained are reacted with polymethyl methacrylate or polycarbonate.

10. A photosensitizer comprising at least one phthalocyanine derivative of general formula I according to claim 1 and/or of general formula II according to claim 2 in an amount of from 0.1 to 10% by weight.

11. The photosensitizer according to claim 10, **characterized in that** said phthalocyanine derivative of general formula I and/or II is present in a form covalently bonded to a polymer matrix.

12. The photosensitizer according to claim 11, **characterized in that** said polymer matrix comprises fibers, granules, powders, non-woven, woven or knitted fabrics or shaped bodies, especially membranes.

13. Use of photosensitizers according to one or more of claims 10 to 12 for initiating photochemical reactions by the action of visible light.

14. The use of photosensitizers according to claim 13 for the catalytic formation of singlet oxygen from triplet oxygen by the action of visible light.

15. The use of metallated and/or unmetallated phthalocyanines bound to polymers according to claim 13 for the detoxification and/or disinfection of oxygen and especially water or water-containing environments by irradiation with light, especially actinic light.

16. The use according to claim 13 for the processing of service water, especially in water cycles; as intermediary purification or final purification in industrial or communal waste water treatment plants; for the purification of washing water in the food industry, especially in the washing of fruits and vegetables prior to canning; for keeping water storage tanks clean; for drinking water stabilization; in combination with gas scrubbers in which loaded washings are obtained; in the treatment of washing, bathing and showering water of industrial and communal plants, especially hotels or camping sites; for the purification of swimming pool waters, especially in combination with solar water heating; for the purification of exhaust air or circulated air, especially of air from cattle sheds, or for deodorizing exhaust air or recirculated air streams in buildings, for the disinfection and/or detoxification of all kinds of objects.

17. The use according to claim 13, **characterized in that** said phthalocyanines are used as stationary structures, . especially sheets of solid material or coated sheets, and as granules, powders, membranes or filters.

18. The use according to claim 13, **characterized in that** phthalocyanines are employed in the form of flat-bed reactors or bubble columns.

19. Containers or articles for the detoxification and/or disinfection of objects, water or water-containing environments, comprising surface regions made of phthalocyanine derivatives bound to a polymer according to claim 1.

20. The container according to claim 19, comprising purification devices for contact lenses, dental prostheses or dental regulatory devices.

21. The articles according to claim 19, comprising antibacterial coatings of surfaces in medical engineering.

## Revendications

1. Phtalocyanine métallée ou non-métallée de formule générale I 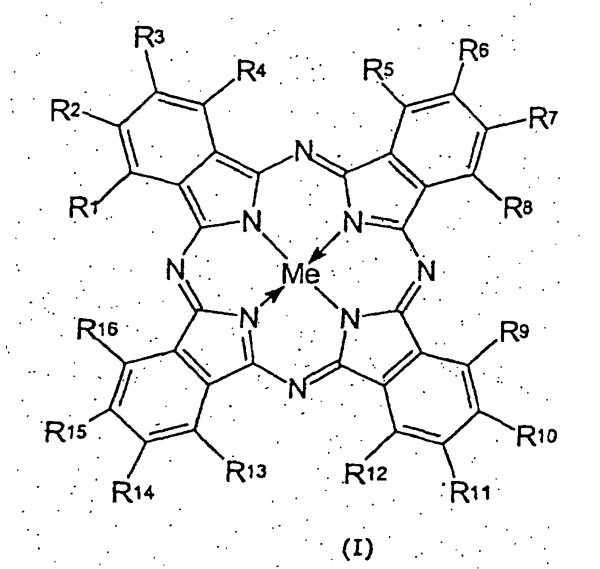 où R¹ à R¹⁶ représentent éventuellement une partie d'un noyau benzocondensé contenant éventuellement de l'azote, portant des substituants R¹' à R¹⁶', R' à R¹⁶, ou bien R¹' à R¹⁶', représentent chacun un groupe hydroxyle ou un reste de polymère relié par C-O-C, le reste de polymère étant issu de polyesters, de poly(méthacrylate de méthyle), de poly(acide méthacrylique), poly(acide acrylique) ou leurs esters, de poly(méthacrylamide), de polyacétals, de polyimides et/ou de polyamides, et englobant aussi leurs constituants monomères, ou bien représentent un atome d'hydrogène, un groupe alkyle, aryle, aralkyle ou alkylaryle, un groupe alkyl-, aryl-, aralkyl- ou alkylarylsulfonate, ou encore SO₃H, F, Cl, ou un groupe pyridin-N-alkyle, -aryle, -alkylaryle ou -aralkyle, le groupe alkyle, aryle, alkylarylke ou aralkyle ayant à chaque fois 1 à 8 atomes de carbone, étant entendu qu'exactement l'un des restes R¹ à R¹⁶ ou R¹' à R¹⁶' représente un groupe hydroxyle ou un reste de polymère relié par C-O-C, et qu'au moins l'un des restes R¹ à R¹⁶ ou R¹' à R¹⁶' représente un groupe SO₃H, et Me représente un cation métallique ou la saturation avec des atomes d'hydrogène.

2. Phtalocyanine métallée ou non-métallée de formule générale II 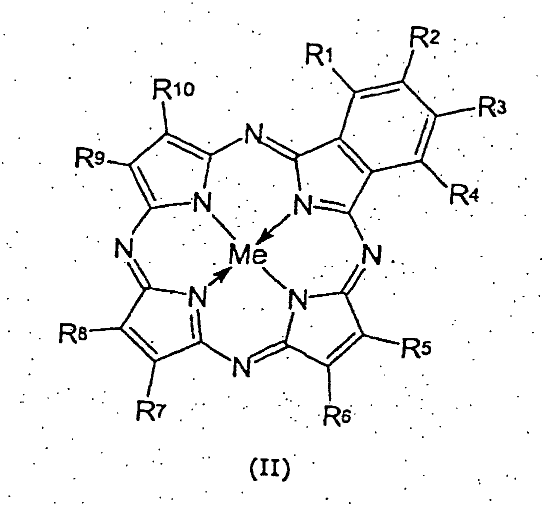 dans laquelle R¹ à R¹⁰ représentent éventuellement une partie d'un noyau benzocondensé contenant éventuellement de l'azote, portant des substituants R¹' à R¹⁰', R¹ à R¹⁰, ou bien R¹' à R¹⁰', représentent chacun un groupe hydroxyle ou un reste de polymère relié par C-O-C, le reste de polymère étant issu de polyesters, de poly(méthacrylate de méthyle), de poly(acide méthacrylique), poly(acide acrylique) ou leurs esters, de poly(méthacrylamide), de polyacétals, de polyimides et/ou de polyamides, et englobant leurs constituants monomères, ou bien représentent un atome d'hydrogène, un groupe alkyle, aryle, aralkyle ou alkylaryle, un groupe alkyl-, aryl-, aralkyl- ou alkylarylsulfonate, ou encore SO₃H, F, Cl, ou un groupe pyridin-N-alkyle, -aryle, -alkylaryle ou -aralkyle, le groupe alkyle, aryle, alkylaryle ou aralkyle ayant à chaque fois 1 à 8 atomes de carbone, étant entendu qu'exactement l'un des restes R¹ à R⁴ représente un groupe hydroxyle ou un reste de polymère relié par C-O-C, qu'au moins l'une des paires de groupes R⁵/R⁶, R⁷/R⁸ et R⁹/R¹⁰ représente un noyau benzocondensé contenant de l'azote, portant des substituants correspondants R⁵' à R¹⁰', l'azote étant alkylé, arylé, alkylarylé ou aralkylé par un groupe ayant à chaque fois 1 à 8 atomes de carbone, et qu'au moins l'un des restes R¹ à R¹⁰ ou R¹' à R¹⁰' représente un groupe SO₃H, et Me représente un cation métallique ou la saturation avec de l' hydrogène.

3. Phtalocyanine métallée ou non-métallée de formule générale I ou II, dans laquelle R¹ à R¹⁶, ou bien R¹ à R¹⁰, représentent de l'hydrogène, étant entendu qu'exactement l'un des restes R¹ à R¹⁶ ou R¹ à R⁴ représente un groupe hydroxyle.

4. Dérivés métallés de phtalocyanine selon la revendication 1 et/ou 2, pour lesquels Me représente Zn ou Al.

5. Procédé de préparation de dérivés de phtalocyanine de formule générale I et/ou II selon la revendication 1 et/ou 2, contenant un constituant monomère d'un reste de polymère, lié par C-O-C, par réaction d'acide phtalique éventuellement substitué, d'acide hydroxyphtalique et d'urée, suivie d'une polymérisation.

6. Procédé de préparation de dérivés de phtalocyanine de formule générale I ou II selon la revendication 1 et/ou 2, contenant un constituant monomère d'un reste de polymère, lié par C-O-C, par réaction d'acides 2,3-naphtalènedicarboxyliques, d'acide hydroxyphtalique et d'urée, suivie d'une polymérisation.

7. Procédé de préparation de dérivés de phtalocyanine de formule générale I et/ou II, selon la revendication 1 et/ou 2, contenant un constituant monomère d'un reste de polymère, lié par C-O-C, par réaction d'acide pyridinedicarboxylique éventuellement substitué, d'acide hydroxyphtalique et d'urée, suivie d'une polymérisation.

8. Procédé selon l'une quelconque des revendications 5 à 7, **caractérisé en ce que** l'on fait réagir le dérivé de phtalocyanine de formule générale I ou II avec un agent de sulfonation, en particulier l'acide chlorosulfonique ou l'acide sulfurique concentré.

9. Procédé de préparation de dérivés de phtalocyanine de formule générale I selon la revendication 1 et/ou de formule générale II selon la revendication 2, par réaction d'acide hydroxyphtalique et d'urée avec un acide phtalique éventuellement substitué, en particulier l'acide 2,3-naphtalènedicarboxylique, **caractérisé en ce que** l'on fait réagir les dérivés de phtalocyanine obtenus, contenant des groupes hydroxyle, avec un poly(méthacrylate de méthyle) ou un polycarbonate.

10. Photosensibilisateur qui comprend au moins un dérivé de phtalocyanine de formule générale I selon la revendication 1 et/ou de formule générale II selon la revendication 2, en une proportion de 0,1 à 10 % en poids.

11. Photosensibilisateur selon la revendication 10, **caractérisé en ce que** le dérivé de phtalocyanine de formule générale I et/ou II est présent sous une forme liée de manière covalente à une matrice de polymère.

12. Photosensibilisateur selon la revendication 11, **caractérisé en ce que** la matrice de polymère comprend des fibres, des granulés, une poudre, des toisons, des tissus, des tissus à maille, des tricots ou des pièces moulées, en particulier des membranes.

13. Utilisation des photosensibilisateurs selon l'une quelconque des revendications 10 à 12, pour le déclenchement de réactions photochimiques par action de la lumière visible.

14. Utilisation de photosensibilisateurs selon la revendication 13, pour la formation catalytique d'oxygène singulet à partir d'oxygène triplet par action de la lumière visible.

15. Utilisation de phtalocyanines liées à un polymère, métallées et/ou non-métallées, selon la revendication 13, pour la désintoxication et/ou la désinfection de l'oxygène et en particulier de l'eau ou d'environnements contenant de l'eau, par irradiation avec de la lumière, en particulier de la lumière actinique.

16. Utilisation selon la revendication 13, pour le traitement de l'eau industrielle, en particulier dans des circuits d'eau, comme purification intermédiaire ou purification finale dans des installations de clarification industrielles ou communales, pour la purification de l'eau de lavage dans l'industrie alimentaire, en particulier pour le lavage des fruits et des légumes avant la conservation, pour le maintien de la pureté des réservoirs d'eau, pour la stabilisation de l'eau potable, en combinaison avec des laveurs de gaz où sont produites des eaux de lavage chargées, pour le traitement de l'eau de lavage, de bain et de douche d'installations industrielles et communales, en particulier les hôtels et les terrains de camping, pour la purification de l'eau des piscines, en particulier en combinaison avec le chauffage solaire de l'eau, pour la purification de l'air sortant ou de l'air circulant, en particulier de l'air des étables, ou pour la désodorisation des courants d'air sortant et d'air circulant dans des bâtiments, et pour la désinfection et/ou la désintoxication d'objets de toutes sortes.

17. Utilisation selon la revendication 13, **caractérisée en ce que** l'on emploie la phtalocyanine sous la forme de structures fixes, en particulier de plaques en matériau plein ou de plaques revêtues, ou sous la forme de granulés, de poudre, de membrane ou de filtre.

18. Utilisation selon la revendication 13, **caractérisée en ce que** l'on emploie la phtalocyanine sous la forme de réacteurs à lit plat ou de réacteurs à colonnes à bulles.

19. Récipients ou objets pour la désintoxication et/ou la désinfection d'objets, de l'eau ou d'environnements contenant de l'eau, qui comprennent des domaines superficiels en dérivés de phtalocyanine liés à un polymère selon la revendication 1.

20. Récipients selon la revendication 19, qui comprennent des appareils de purification pour les lentilles de contact, les prothèses dentaires ou les dispositifs de réglage des dents.

21. Objets selon la revendication 19, qui comprennent des revêtements antibactériens de surfaces en technique médicale.
